# EUROPEAN PATENT APPLICATION

(11) **EP 3 446 637 A1**
(43) Date of publication of application: **27.02.2019**
(21) Application number: 17775204.5
(22) Date of filing: 29.03.2017
(51) Int. Cl.: A61B 17/06

(54) **SUTURE NEEDLE**

(30) Priority: 31.03.2016 JP 2016071200
(71) Applicant: SMR Co., Ltd., Tokyo 108-0072 (JP)
(72) Inventor: MATSUMOTO, Keisuke, Tokyo 108-0072 (JP)
(74) Representative: J A Kemp
(86) International application number: PCT/JP2017/012866
(87) International publication number: WO 2017/170670

(57) **Abstract**

The surgical suture needle (10) includes a tip section (12), a main body (14), and a base end portion (16). The tip section (12) is colored in a color different from a color of other portions (14, 16). The tip section (12) has a tapered shape, and a length of the tip section (12) is 10% of a whole length of the suture needle (10). When the suture needle (10) penetrating the biological object (30) is pulled out by grasping with a needle holder (28), grasping the tip section (12) of the suture needle (10) easily breaks the suture needle (10). Therefore, a doctor grasps a portion (14) other than the tip section (12) with the needle holder (28). The colored tip section (12) ensures the doctor easily distinguishing the tip section (12) from the other portions (14, 16). Coloring the tip section (12) in red, green, purple, or pink ensures avoiding erroneously grasping the tip section (12) since the tip section (12) is unnoticeable at a suture position where blood is attached.

## Description

### TECHNICAL FIELD

The present invention relates to a suture needle.

### BACKGROUND ART

JP2015-58112A discloses a suture needle for the purpose of facilitating distinguishing a front and a back, and a direction of a suture needle. In the suture needle, a first line and a second line are formed on an outer peripheral side and an inner peripheral side, respectively, of the suture needle whose entire shape is curved, along a longitudinal direction of the suture needle. Furthermore, a third line that surrounds a circumferential direction of the suture needle is formed. A plurality of the third lines are formed at constant intervals along the longitudinal direction of the suture needle.

When a suture needle is used, the following operation is typically performed: the suture needle is grasped with a needle holder, for example, a tip of the suture needle is pierced from a skin of a patient (a biological object) toward a subcutaneous tissue (a biological object), the suture needle is passed through the subcutaneous tissue following a curved shape of the suture needle such that the tip of the suture needle returns to a side of the skin where pierced, and the tip section exposed from the skin is grasped with the needle holder and the suture needle is pulled out from the subcutaneous tissue and the skin.

### SUMMARY OF INVENTION

However, when the suture needle is grasped at the tip section, and then pulled out, the tip section may be subjected to a load. To avoid this, it is necessary to pull out the suture needle, not by grasping the tip section with the needle holder, but by grasping a further base end side (a side where a suture thread is threaded through) with respect to the tip section. Forming the third lines on the suture needle as in Patent Literature 1 and using this as a mark ensure avoiding the tip section from being grasped, but there still remains a problem of erroneously grasping the tip section since its distinctiveness as a mark is not high.

Then, the present invention focuses on the above-described problem, and it is an object of the present invention to provide a suture needle that reminds a doctor who performs surgery not to grasp a tip section of the suture needle to ensure reducing a load to the tip section of the suture needle.

In order to achieve the above-described object, a suture needle according to the present invention has a tip section of the suture needle made by being colored in a color different from a color of a portion other than the tip section.

### BRIEF DESCRIPTION OF DRAWINGS

FIG. 1 is a schematic diagram of a suture needle of a first embodiment and a partially enlarged view thereof.
FIG. 2 is a schematic diagram of an operation to suture a biological object using the suture needle of the first embodiment (before piercing).
FIG. 3 is a schematic diagram of the operation to suture the biological object using the suture needle of the first embodiment (after piercing and before exposure of tip).
FIG. 4 is a schematic diagram of the operation to suture the biological object using the suture needle of the first embodiment (after exposure of tip).
FIG. 5 is a schematic diagram of the operation to suture the biological object using the suture needle of the first embodiment (before pulling out).
FIG. 6 is a schematic diagram of the operation to suture the biological object using the suture needle of the first embodiment (after pulling out).
FIG. 7 is a drawing illustrating a distribution of broken positions after use in a suture needle curved into a circular arc shape.
FIG. 8 is a partially enlarged view of a suture needle of a second embodiment.
FIG. 9 is a partially enlarged view of a suture needle of a third embodiment.

### DESCRIPTION OF EMBODIMENTS

The following describes embodiments of the present invention with reference to the drawings.

### [First Embodiment]

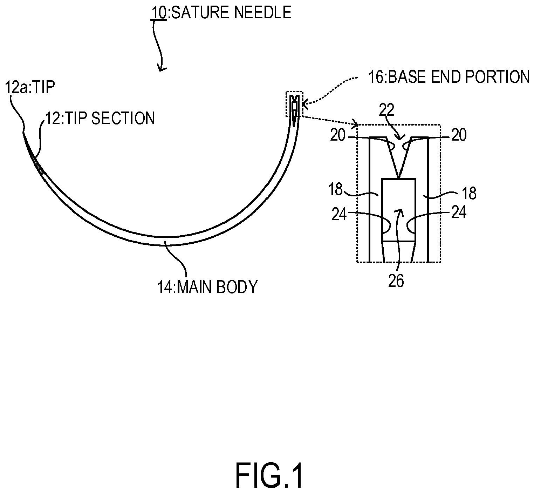

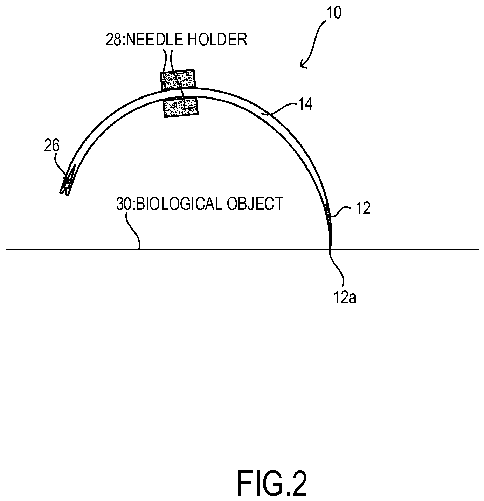

FIG. 1 illustrates a schematic diagram of a suture needle of a first embodiment and a partially enlarged view thereof. A surgical suture needle 10 of this embodiment uses, for example, stainless steel as a material, and has an outer shape curved into an approximately semicircle. The suture needle 10 is configured of a tip section 12, a main body 14, and a base end portion 16. Cross-sectional shapes of the tip section 12, the main body 14, and the base end portion 16 are not particularly limited, and, for example, a circular shape and a polygon shape, such as a triangular shape, are applicable. This cross-sectional shape can be designed to change as moving in a longitudinal direction of the suture needle 10. It should be noted that the suture needle 10 has a portion up to approximately 1/3 of the length of the suture needle 10 including the tip section 12, and the portion serves as an edge that pierces into a biological object 30 (see FIG. 2).

The tip section 12 is a portion having a tip 12a that is first pierced into the biological object 30, of the suture needle 10. The tip section 12 has a tapered shape whose diameter decreases toward the tip 12a. The main body 14 can also have a tapered shape whose diameter decreases toward a side of the tip section 12 from a middle in the longitudinal direction (direction along the circular arc shape of the suture needle 10) of the suture needle 10. The base end portion 16 is a portion where a suture thread (not illustrated) is inserted (held) through. The base end portion 16 has a pair of arms 18 extending along the longitudinal direction of the suture needle 10. The pair of arms 18 have inclined surfaces 20 at the tip side on side surfaces on sides facing one another. With the two inclined surfaces 20, a clearance between the pair of arms 18 forms a wedge-shaped introduction portion 22. The pair of arms 18 have respective cutouts 24 formed at a base side on side surfaces on the sides facing one another. The two cutouts 24 facing one another form a through-hole 26 through which the suture thread is inserted (held) through.

When the suture thread is threaded through the through-hole 26, simply pressing side surfaces of the suture thread onto the introduction portion 22 causes its force temporarily to bend the pair of arms 18 in a direction where the pair of arms 18 separate from one another for the suture thread to pass between the pair of arms 18, and then, the suture thread is directly introduced into the through-hole 26. Obviously, an end portion of the suture needle 10 may be directly inserted through the through-hole 26 without using the introduction portion 22. It should be noted that the suture needle includes a "suture thread with needle," which is a preliminarily joined suture needle and suture thread, and a "medical suture needle," which is used by combining a suture needle and a suture thread when it is used.

This embodiment is characterized in that the tip section 12 of the suture needle 10 is colored in a color different from a color of a portion other than the tip section 12. Coloring method includes methods, such as applying a pigment and/or a dye harmless to human body, chemically/ electrochemically plating metal or the like harmless to human body, and forming an oxide film (color is visible by interference of light) on a surface. With the pigment and/or the dye, a desirable color can be selected; for example, red (or a warm color, such as orange), a neutral color, such as green, and a chromatic color, such as purple and pink, are applicable. With plating, a color unique to metal that is plated is usable. In the case of the oxide film, colors, such as black, green, blue, gold, and magenta (a reddish color), are selectable.

Meanwhile, the main body 14 and the base end portion 16 are not necessarily colored in particular. In a case of being colored, it is only necessary that the main body 14 and the base end portion 16 are colored in a color different from that of the tip section 12, and for the coloring method, for example, the above-described application, plating, and forming of oxide film can be performed as desired.

Next, an operation to suture the biological object 30 using the suture needle 10 of the embodiment will be described with reference to FIG. 2 to FIG. 6. It should be noted that the suture thread is omitted from the illustration also in FIGS. 2 to 6.

As illustrated in FIG. 2, the suture needle 10 having the suture thread inserted through the through-hole 26 is grasped with a needle holder 28 (only a grasping portion is illustrated) and the tip 12a of the suture needle 10 is brought into contact with a suture position of the biological object 30 at a predetermined angle.

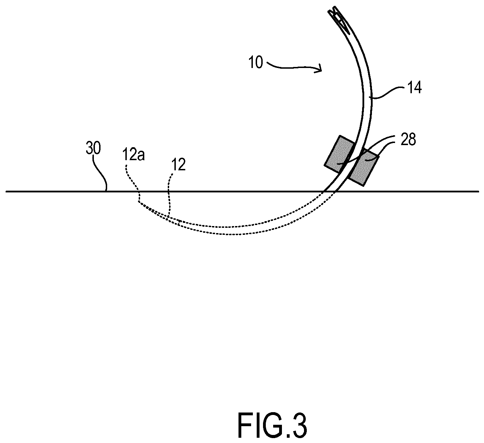

As illustrated in FIG. 3, the tip 12a of the suture needle 10 is pierced into the biological object 30 using the needle holder 28, and a piercing operation of the suture needle 10 is advanced with the grasping angle of the suture needle 10 with the needle holder 28 being changed following a curved shape of the suture needle 10. Here, when the suture needle 10 is pierced into the biological object 30, it is preferred that a direction of the suture needle 10 is operated such that the tip section 12 is pierced into the biological object 30 in an approximately perpendicular direction. In this embodiment, while the needle holder 28 is applied as a tool to grasp the suture needle 10, another grasping means, such as tweezers, is applicable.

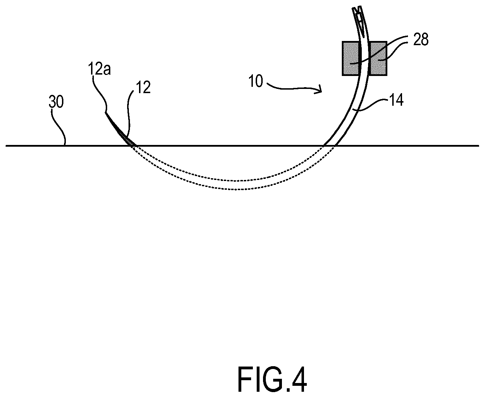

As illustrated in FIG. 4, the piercing operation of the suture needle 10 is further advanced. Then, while the tip 12a and the tip section 12 are exposed from the biological object 30, a pulling out operation of the suture needle 10 cannot be performed yet since the tip section 12 is a portion where grasping is inhibited.

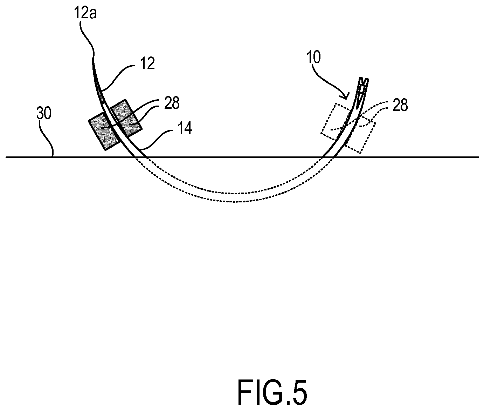

As illustrated in FIG. 5, when the piercing operation of the suture needle 10 is further advanced, the main body 14 of the suture needle 10 is exposed from the biological object 30. This allows the needle holder 28 to change the grasping position from the base end portion 16 side to a position adjacent to the tip section 12 of the main body 14, and then, the pulling out operation can be performed.

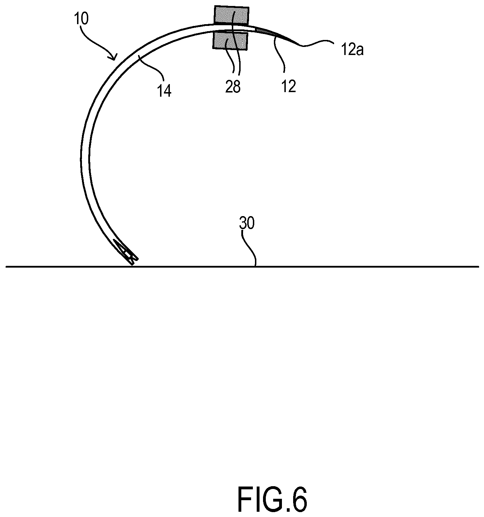

As illustrated in FIG. 6, the pulling out operation of the suture needle 10 is advanced while an angle of grasping the suture needle 10 with the needle holder 28 is changed following the curved shape of the suture needle 10. At that time, since the needle holder 28 does not grasp the tip section 12 of the suture needle 10, no load is applied to this tip section 12. Finally, completely pulling out the suture needle 10 from the biological object 30 sutures the biological object 30 with the suture thread.

With the suture needle 10 according to the embodiment, it is easy to distinguish the tip section 12 from another portion since the tip section 12 where grasping is inhibited is colored in a different color from a color of another portion. This can call an attention of a doctor who performs a surgery, and thus, grasping the tip section 12 of the suture needle 10 is avoided to reduce a load to the tip section 12.

In particular, it is preferable to color the tip section 12 in red in this embodiment. During surgery, the suture position has a blood of a patient attached to have a dominant color of the blood. Therefore, coloring the tip section 12 in red makes the tip section 12 unnoticeable, thus ensuring avoiding the tip section 12 from being erroneously grasped. It should be noted that, in a surgery site, it is necessary to maintain an environment where the blood is always distinguishable from an aspect of, for example, infection prevention. Therefore, red is not usually used in a medical apparatus. However, the used suture needle 10 is always removed, and then, sterilized or disposed, therefore not generating a problem of, for example, an infection. The tip section 12 being colored in purple and pink, which are colors similar to red, has a similar effect.

It is also preferable to color the tip section 12 green. This ensures distinguishing the tip section 12 before the tip section 12 is pierced into the biological object 30 but when this tip section 12 is exposed from the biological object 30, the blood is attached to the tip section 12 to show the tip section 12 in a color close to black, thus ensuring the tip section 12 becoming unnoticeable.

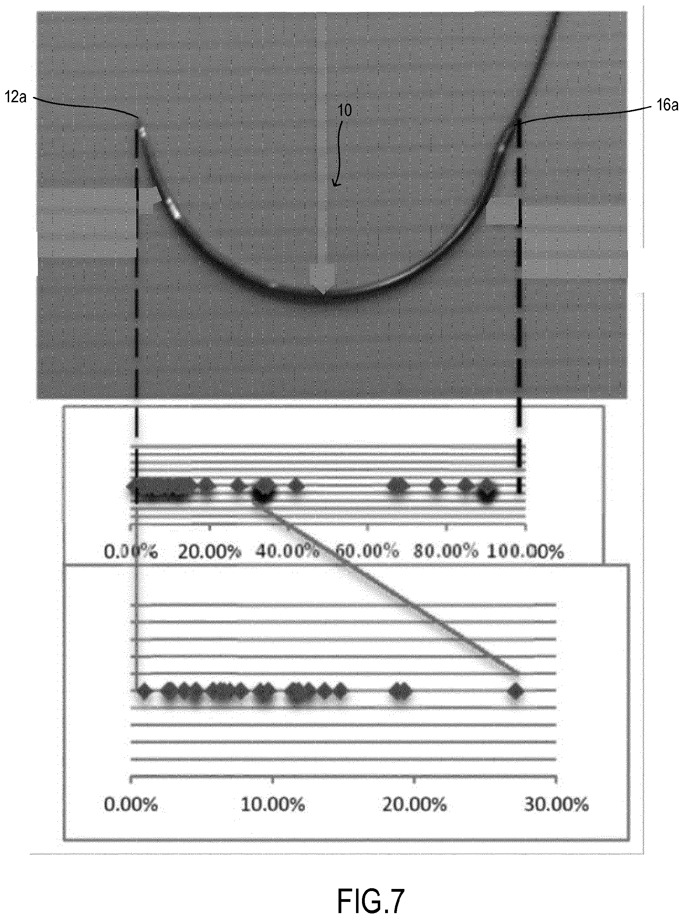

The inventor of the present application collected a large number of suture needles broken during suturing operations to examine a distribution of the broken positions. FIG. 7 illustrates the distribution of the broken positions in the suture needle 10, which is curved into a circular arc shape, with a position of the tip 12a being 0 percent and a position of a base end 16a being 100 percent. While the suturing operation with the used suture needle has an aspect similar to the aspects illustrated in FIG. 2 to FIG. 6, there also are cases where the tip section 12 is grasped. As illustrated in FIG. 7, it is seen that the broken positions of the suture needle 10 are concentrated from the tip 12a of the suture needle 10 to a position at 10 percent in the longitudinal direction of the suture needle 10. This is considered that the suture needle 10 broke due to a concentrated stress to the position at 10 percent caused by grasping the tip 12a side with respect to this position at 10 percent when the suture needle 10 is pulled out from the biological object 30 (see FIG. 5). As illustrated in FIG. 7, it is seen that the distribution is sparse and a count of the broken suture needle 10 is significantly decreased in the base end 16a side with respect to a position at 15 percent in the longitudinal direction.

Accordingly, from the result illustrated in FIG. 7, it is considered preferable to color up to a portion at a length up to 10 percent (or 15 percent) including the tip 12a of the suture needle 10 in the longitudinal direction of the suture needle 10 as the tip section 12 (see FIG. 1). This calls an attention of the doctor who performs the surgery to avoid grasping this tip section 12 and avoid the stress applied to the suture needle 10 from concentrating on a certain portion of the suture needle 10 when the suture needle 10 is pulled out, and thus, the load to the suture needle 10 can be reduced.

In this embodiment, the pair of arms 18 that form the through-hole 26 are disposed in the base end portion 16, the through-hole 26 may be a penetration hole that simply penetrates the base end portion 16. It may be configured to be a "suture thread with needle" in which the suture thread is joined to the base end portion 16 with an adhesive agent or the suture thread is coupled to the base end portion 16 by crimping or the like. Furthermore, in this embodiment, the suture needle 10 has an approximately semicircle circular arc shape but the suture needle 10 may have a linear shape. Even with the circular arc shape, a shape other than the semicircle circular arc, for example, circular arc shapes in aspects extracted at respective ratios of lengths of 5/8, 7/16, 3/8, and 1/4 from, for example, a circular arc (ring) of the whole circumference are also applicable. Furthermore, one that is rectilinear in whole but has only the tip section curved into a circular arc shape is also applicable. It should be noted that with any suture needle, a suturing procedure is performed similarly to FIG. 2 to FIG. 6.

### [Second Embodiment]

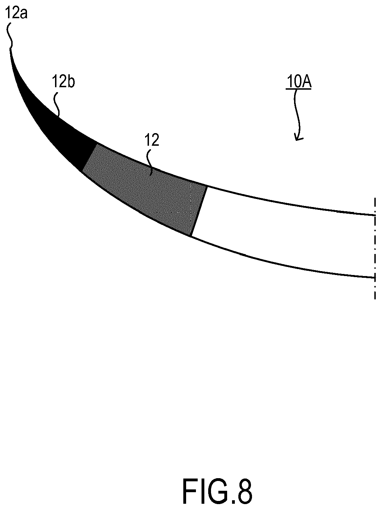

FIG. 8 is a partially enlarged view of the suture needle in a second embodiment. As illustrated in FIG. 8, a suture needle 10A according to the second embodiment has an outer shape in common with the suture needle 10 of the first embodiment. The tip section 12 has a tip region 12b from the tip 12a of the suture needle 10A to a midpoint in the longitudinal direction of the tip section 12. The tip region 12b is colored in a color different from a color of a portion other than that (tip region 12b) of the tip section 12. In particular, the tip region 12b is preferred to be colored in black, or may be colored in white, gold, and silver, other than black.

Thus, coloring the tip region 12b in a color different from a color of another portion of the tip section 12 causes the doctor to visually recognize a presence of the tip 12a that exposes again from the biological object 30 after piercing the suture needle 10 into the biological object 30 (not illustrated in FIG. 8), thereby ensuring enhanced operation efficiency of suturing.

### [Third Embodiment]

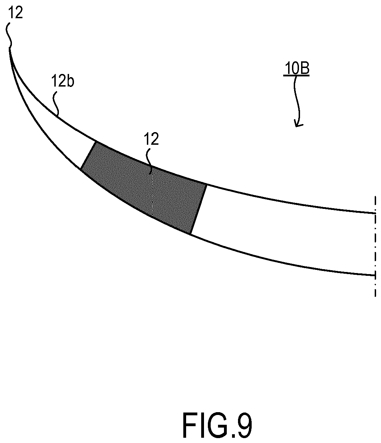

FIG. 9 is a partially enlarged view of a suture needle of a third embodiment. As illustrated in FIG. 9, a suture needle 10B of the third embodiment has an outer shape in common with the suture needle 10 of the first embodiment. The tip region 12b from the tip 12a of the suture needle 10 to the midpoint in the longitudinal direction of the tip section 12 in the tip section 12 is not colored. The tip region 12b is in a state where a surface of the suture needle 10 is exposed and is a portion where a color (for example, silver or brown) of a material of the suture needle 10 appears.

Thus, not coloring the tip region 12b causes the doctor to visually recognize a presence of the tip 12a exposed again from the biological object 30 after piercing the suture needle 10 into the biological object 30 (not illustrated in FIG. 9), thereby ensuring the enhanced operation efficiency of suturing.

In the second embodiment and the third embodiment, a ratio of the length of the tip region 12b to the tip section 12 is preferred to be 50 percent or less. This ensures both calling an attention to the tip 12a and avoiding grasping the tip section 12 at the same time.

The embodiments of the present invention described above merely describes some application examples of the present invention and not of the nature to limit the technical scope of the present invention to the specific constructions of the above embodiment.

This application is based on and claims priority to Japanese Patent Application No. 2016-71200 filed in Japan Patent Office on March 31, 2016, the entire content of which is incorporated herein by reference.

## Claims

1. A surgical suture needle, wherein
the suture needle has a tip section colored in a color different from a color of a portion other than the tip section.

2. The suture needle according to claim 1, wherein
the tip section has a color of any of red, green, purple, and pink.

3. The suture needle according to claim 1 or 2, wherein
the tip section is a portion having a length up to 10 percent in a longitudinal direction of the suture needle, the portion including a tip of the suture needle.

4. The suture needle according to any one of claims 1 to 3, wherein
the tip section has a tip region from a tip of the suture needle to a midpoint in a longitudinal direction of the tip section, the tip region being colored in a color different from a color of a portion other than the tip region of the tip section.

5. The suture needle according to claim 4, wherein
the tip region has a color of black.

6. The suture needle according to any one of claims 1 to 3, wherein
the tip section has a tip region from a tip of the suture needle to a midpoint in a longitudinal direction of the tip section, the tip region being not colored.

7. The suture needle according to any one of claims 4 to 6, wherein
the tip region has a ratio of length to the tip section of 50 percent or less.
